# EUROPEAN PATENT APPLICATION

(11) **EP 4 344 638 A1**
(43) Date of publication of application: **03.04.2024**
(21) Application number: 22198929.6
(22) Date of filing: 30.09.2022
(51) Int. Cl.: A61B 5/259, A61B 5/266

(54) **MEDICAL ELECTRODE FOR LONG-TERM APPLICATIONS**

(71) Applicant: Ambu A/S, 2750 Ballerup (DK)
(72) Inventor: Hansen, Daniel, 2100 Copenhagen (DK); Riis Dammeyer, Marie, 2750 Ballerup (DK)
(74) Representative: Guardian IP Consulting I/S

(57) **Abstract**

The present disclosure relates to a medical electrode for long-term biopotential monitoring and/or recording of biopotential signals, said medical electrode comprising a sensor element, a device backing, an ion conductive layer in contact with the skin side of the sensor element, and a water absorbent skin adhesive layer surrounding the ion conductive layer and having an upper side and a skin side adapted to attach to skin.

## Description

The present disclosure relates to a medical electrode for long-term biopotential monitoring and/or recording of biopotential signals, said medical electrode comprising a sensor element, a device backing, an ion conductive layer in contact with the skin side of the sensor element, and a water absorbent skin adhesive layer surrounding the ion conductive layer and having an upper side and a skin side adapted to attach to skin.

Medical electrodes are used for establishing electrical contact between the skin of humans or animals and electrical measuring equipment for monitoring or recording the bioelectrical signals caused by physiological processes, such as the heart function. Medical electrodes are typically provided with a skin-friendly adhesive that provides attachment to the patient's skin.

Clinical procedures such as telemetry, Holter and event monitoring require continuous acquisition of electrocardiogram (ECG) signals for several days. For successful ECG monitoring, the medical electrodes must be attached to the patient's skin for the entire monitoring period with minimum disturbances of the recorded ECG signal. Increasing the monitoring times emphasize certain requirements specifically related to skin adhesion, skin friendliness, signal acquisition and the electrode's ability to mitigate signal noise.

In long-term applications, which includes monitoring, the patient is equipped with medical electrodes, which are worn for a prolonged period, such as from four days and up to many days. This means that the medical electrode has to remain attached to the patient for the entire wear time, which could be for 4, 5 or even 7 days or more, e.g. up to 10 or 14 days. In such situations, it is important that the medical electrode maintains good adhesive properties to the skin during the whole wear time in order to provide stable and continuous monitoring and/or recording of the biopotential signals. This is particularly challenging when the purpose is monitoring a patient's biopotentials during body movements and sweat secretions during usual daily activities, such as when the patient is walking, running, cycling or exercising. The medical electrode must also be able to withstand and remain attached to the skin, during and after the patient bathes and sleeps.

Water is continuously released from the human body through the skin. Water is released through perspiration (or sweat) and as transdermal water loss (TEWL). TEWL is water vapour passively diffusing across the skin barrier. Perspiration is where liquid water is actively pumped through sweat pores to the skin surface, e.g. during physical activities.

During clinical procedures, where a medical electrode is attached to a patient's skin, water released from the skin may spread at the skin-adhesive interface and often leads to adhesive failure. Prolonged exposure to liquid water, e.g. sweat trapped at the interface between the skin and the medical electrode, may lead to reduced skin barrier properties and cause skin maceration. Reduced skin barrier properties and skin maceration makes the skin much more susceptible to chemical skin irritation and/or infections.

The amount of sweat released from the skin depends on a number of environmental and individual parameters such as temperature, humidity, age and activity level of the patient. For example, the perspiration will be higher during physical exercise, such as running, in comparison to e.g. sedentary office work or other activities where the patient is physically inactive.

It is well recognized that neonates are a special group of patients with special needs, in particular pre-term neonates, because their skin is thin and very delicate and because the neonates are nursed in special environments when hospitalized in Neonatal Intensive care units (ICU) where the neonates are nursed in incubators. Additionally, it is well recognized that neonates and babies are physically inactive and only has transepidermal water loss (TEWL) and do not perspire as adults or older children do, i.e. children with higher physical activity, such as when crawling, walking or running etc.

US 4,524,775 (to Medicotest A/S) discloses a medical electrode for contacting the skin. The medical electrode comprises a foam plastic disc, which is formed with an aperture and the part of the disc surrounding the aperture is covered by a plastic foil on one side thereof. A strip-shaped metal electrode is placed below the foil in the aperture and is connected to the stripped end of a plastic insulated lead, whose end portion is covered by the cover foil. The whole assembly is welded together to form a unit. The foam disc is compressed and fixed in the compressed shape because the cell walls partly fuse. The foam plastics becomes so tight that practically no diffusion can take place through it, without the compression and fusing noticeably increasing the rigidity of the electrode. Thus, a medical electrode is provided which is permeable to sweat in the adhering zone, but form a barrier to moisture in the measuring area.

The electrode disclosed in US 4,524,775 provides good signal quality, has a large measuring area to ensure an optimal signal during stress tests and monitoring applications. The foam disc is made of a soft and pliable material, which makes the electrode easy to use and comfortable to wear, but it is occlusive to liquids and vapour and may thus not be gentle to the skin if used for long-term applications as the occlusive layer may result in skin maceration beneath the foam. The contact medium, i.e. the medium which is used for establishing good electrical connection between the metal electrode and the skin is generally a paste-like electrolyte, i.e. a gel with high water activity, which may cause skin maceration if the medical electrode is applied to the same skin area for a prolonged period, e.g. about more than three days.

GB 2341104 A (to Medicotest A/S) discloses a medical electrode for picking up electrical signals from a person's skin. The electrode comprises a carrier sheet having an Ag/AgCI sensor with a sponge with an electrically conductive gel in contact with the Ag/AgCI sensor and a ring of adhesive foam material with a skin friendly adhesive around the sponge with the gel for attachment to the person's skin. The electrode is particularly useful for recording diagnostic or rest ECG, which is a short-term application, typically few minutes or up to a few hours. The electrically conductive gel is preferably a wet gel, which has been soaked into the sponge. The wet gel gives good electrically contact with the skin, but it has a high water activity and may cause skin maceration if the wet medical electrode is applied to the same skin area for prolonged period, e.g. about more than three days.

CN213606413U discloses an electrode sheet for ECG monitoring with better skin-friendly performance, which is not easy to cause skin allergies, redness and other discomforts for children and patients with high skin sensitivity. The electrode is provided with a hydrocolloid layer, which has the ability to absorb exudate, so when it encounters the patient's sweat or other liquids it has little effect on its adhesiveness. The hydrocolloid layer is provided with vents to increase the air permeability, so that the sweat on the patient's skin can be volatilized through the vent hole. The electrode sheet may further include a liquid absorbing layer laminated with the hydrocolloid layer. It is taught that this liquid absorbing layer is used to quickly absorb the heat or water vapour emitted through the vents, which can quickly keep the skin dry. Neither the water absorption capacity of the hydrocolloid layer nor the wear time of the electrode is mentioned.

CN212939717U discloses a visual electrode for ECG monitoring comprising a transparent hydrocolloid patch. It is taught that the transparent patch makes it convenient to observe the patient's skin condition and thereby overcome the disadvantage of the existing non-woven material of the electrode sheet that is prone to allergies. Neither the water absorption capacity of the transparent hydrocolloid patch nor the wear time of the electrode is mentioned.

US 8,798,709 B1 discloses sensors, which are attachable to the skin of a neonate or infant. The sensor comprises a hydrogel and hydrocolloid, which is said to provide long-term adhesion (several days) to the patient and prevention of fall off prematurely under moist or humid conditions. The water absorption capacity of the combined hydrogel/hydrocolloid is not mentioned and it is not taught how well the sensor may work or adhere to the body when used for long-term monitoring of a patient during everyday use. In particular, there is no discussion of the challenges of body movements and sweat secretions, which are factors of great importance to whether the electrode is suitable for long-term monitoring of patients on the move.

Vermed is a known producer of medical electrodes. Their TenderTrode PlusTM line of hydrocolloid products by Nissha Medical Technologies | Healthcare Solutions is designed for the unique needs of the neonatal market. It is taught on their website (https://hs.nisshamedical.com/en/ecg-electrodes/tendertrode-plus-prewired/) that their products use hydrocolloid instead of aggressive tape adhesives to prevent irritation to tender skin while providing long-term attachment. This, coupled with the use of soft conforming materials, ensures patient comfort and product effectiveness in the neonatal intensive care use. It is said that the use of hydrocolloid materials over adhesives minimizes the risk of epidermal stripping. The water absorption capacity of the hydrocolloid materials is not mentioned, and there is no hints or suggestions that this property could become important if the electrode is used for another application than the neonatal use, such as for example for long-term monitoring of a patient during every day use. In particular, there is no discussion of the challenges of body movements and sweat secretions, which are factors of great importance to whether the electrode is suitable for long-term monitoring of patients on the move.

The object of the present disclosure is to provide a medical electrode for long-term applications, in particular for long-term biopotential monitoring and/or recording of biopotential signals. The medical electrode is specifically targeted at patients who need long-term monitoring while maintaining their everyday life on the go, which includes toddlers, children and adults.

In view of this object, a medical electrode comprising a water absorbent skin adhesive is provided. The water absorbent skin adhesive has a total water absorption capacity of at least 200 mg water/cm².

In this way a long-term wear time of at least 4 days is obtained. The high water absorption capacity of the skin adhesive absorbs the amount of water released from the skin due to TEWL and perspiration thereby transporting the water away from the skin-adhesive interface. In this way the adhesive strength is maintained and improved during periods with perspiration and thus also in long-term applications. In addition, problems relating to skin maceration and/or irritation is prevented or minimized.

### Water absorbent skin adhesive

Water released from the skin (TEWL and perspiration) may spread at the interface between the skin and the adhesive, which often leads to adhesive failure. Prolonged exposure of the skin to water trapped in the interface between the skin and the adhesive may also lead to reduced skin barrier properties and cause skin maceration. Therefore, there is a need for transporting water away from the skin/adhesive interface. This problem is solved according to the disclosure by using a water absorbent skin adhesive, which can absorb the water (TEWL and perspiration) from the skin. This has shown to help maintain or improve the adhesive strength during periods with perspiration and thus also in long-term applications.

The water absorbent skin adhesive preferably comprises hydrocolloids. Hydrocolloid adhesives are typically rubber-based pressure sensitive adhesives, which comprises colloid particles suspended or embedded in the polymer matrix. Acrylic-based hydrocolloid adhesives are also commercial available. The hydrocolloid particles allow the adhesive to absorb fluid until they become saturated. Hydrocolloid adhesives have different water absorption capacities, as they will absorb fluid until they become saturated. When the hydrocolloid adhesive becomes saturated, it forms a soft, moist gel, but may then loose at least some of its adherence to the skin.

For long-term medical electrodes, a high water absorption capacity is essential to ensure sufficient attachment to the skin throughout the wear period, and to ensure the skin to stay healthy.

The inventors of the present disclosure have surprisingly found that when applying a hydrocolloid skin adhesive with a total water absorption capacity of at least 200 mg water/cm² sufficient attachment throughout a wear time of at least 4 days is obtained. When a wear time of at least 7 days is aimed at, the total water absorption capacity of the hydrocolloid skin adhesive must be at least 350 mg water/cm².

The water absorption capacity, sometimes called total water absorption (the two terms are used interchangeably in this disclosure), is determined by the uptake of water per cm² over a 24-hour period where the hydrocolloid adhesive is immersed in an (isotonic) saline solution of 0.9% (by weight) sodium chloride (NaCI) in water.

The medical electrode may be constructed having various wear times. A specific wear time of the medical electrode demands a minimum total water absorption capacity. The longer wear time, the higher total water absorption capacity is required. For example, the total water absorbent capacity of the water absorbent skin adhesive in the medical electrode is:
- at least 200 mg water/cm² during the first four days of wear time, or
- at least 250 mg water/cm² during the first five days of wear time, or
- at least 300 mg water/cm² during the first six days of wear time, or
- at least 350 mg water/cm² during the first seven days of wear time, or
- at least 400 mg water/cm² during the first eight days of wear time, or
- at least 450 mg water/cm² during the first nine days of wear time, or
- at least 500 mg water/cm² during the first ten days of wear time, or
- at least 550 mg water/cm² during the first eleven days of wear time, or
- at least 600 mg water/cm² during the first twelve days of wear time, or
- at least 650 mg water/cm² during the first thirteen days of wear time, or
- at least 700 mg water/cm² during the first fourteen days of wear time.

The polymer matrix of the hydrocolloid skin adhesive may comprise a number of different polymers, which are commonly known by a skilled person. The polymer in the polymer matrix may be selected from the group consisting of polyisobutylene, styrene-isoprene-styrene, ethylene-vinyl acetate, butyl rubber, acrylate and silicone and any mixture thereof.

The hydrocolloid skin adhesive comprises hydrocolloids. The hydrocolloids are water absorbent particles, which are suspended and/or embedded in the polymer matrix. The hydrocolloid particles in the hydrocolloid skin adhesive may be selected from the group consisting of carboxymethyl cellulose, hydroxyethyl cellulose, cetyl hydroxyethyl cellulose, polyacrylic acid, pectin, locust bean gum, gum karaya and guar gum and any mixtures thereof.

The amount of hydrocolloid particles in the hydrocolloid skin adhesive is typically at least 20 wt% based on total weight of the hydrocolloid skin adhesive, such as in the range of 20-60 wt% based on total weight of the hydrocolloid skin adhesive.

Commercially available hydrocolloid skin adhesives, which typically are developed for use in wound dressings or in ostomy applications, may be used in the present disclosure. Suitable examples include those from Avery Dennison, such as those sold under the tradenames MED 5577A, MED 5094H, MED 2171H, MED 5541H, MED 5589H, MED 5583H, MED 5542H and MED 2190H.

The thickness of the water absorbent skin adhesive layer is typically in the range of 150 to 1500 microns, preferably in the range of 300 to 1000 microns.

The water absorbent adhesive may further comprise an occlusive backing on the upper side, i.e. on the side facing away from the skin. The occlusive backing may be a separate backing layer attached to the hydrocolloid skin adhesive layer. Alternatively or additionally, the backing on the water absorbent skin adhesive layer may be composed of the electrode backing, which also covers the central area of the electrode with the sensor element and the ion conductive layer. Examples of suitable device backings and/or backings on the water absorbent skin adhesives are films of polyethylene terephthalate (PET), polyethylene (PE), polypropylene (PP), ethylene vinyl acetate (EVA), which is a copolymer of ethylene and vinyl acetate, polyvinylidene dichloride (PVDC) or any blend or mixture thereof. The backing may also be a laminate comprising one or more layers, where at least one layer is made of any one of the above-mentioned polymers. The occlusive backing for the hydrocolloid skin adhesive layer may be covered with a woven or non-woven fibrous layer, e.g. of polyethylene terephthalate (PET), polyethylene (PE), polypropylene (PP) or any blend or mixture thereof, to provide a soft surface, which may cause less mechanical stress on the skin surfaces in contact with the upper side of the medical electrode.

The water absorbent skin adhesive layer may have chamfered/beveled face at the circumference to reduce any sharp edges, which could be caught by clothing, itch or scratch surround skin. The beveled edges are believed to reduce the risk of edge lifting.

The opening in the water absorbent skin adhesive layer may have a diameter of 10-30 mm, such as 14-24 mm or preferably 16-22 mm, to provide room for the sensor element and the ion conductive layer. The outer diameter of the water absorbent skin adhesive layer may be in the range of 25 mm to 55 mm or preferably 30-45 mm.

### Rim skin adhesive

The medical electrode may further comprise a rim skin adhesive extending beyond a circumference of the water absorbent skin adhesive layer in order to reduce edge lifting. The rim skin adhesive is a different type of skin adhesive than the water absorbent skin adhesive. Optionally, the rim skin adhesive overlaps at least part of the upper side of the water absorbent skin adhesive. The rim skin adhesive may fully overlap the upper side of the skin adhesive by forming an opening in the rim skin adhesive and aligning this opening with the opening in the water absorbent skin adhesive layer about the alignment axis.

The rim skin adhesive may be an acrylic based adhesive designed for medical and/or surgical use, which includes a pressure sensitive adhesives (PSA), e.g. by selecting an acrylic adhesive from the pool of acrylic adhesives, which are commonly used as skin adhesives in medical electrodes and/or for medical or surgical applications. Additionally or alternatively, the rim skin adhesive may be a hydrocolloid adhesive, e.g. an acrylic skin adhesive comprising hydrocolloid particles, for example with beveled edges. The rim skin adhesive may be selected to have a more tacky property than the water absorbent skin adhesive.

The rim skin adhesive comprises a backing. The backing may be of the same material as the backing on the water absorbent skin adhesive and/or the device backing or a different type of material. The backing on the rim skin adhesive does not need to be occlusive. Examples of suitable separate backings on the rim skin adhesives are films of polyethylene terephthalate (PET), polyolefins, such as polyethylene (PE), low density PE (LDPE), polypropylene (PP) or polyurethane (PU) or any blend or mixture thereof. The backing may also be a laminate comprising one or more layers, where at least one layer is made of any one of the above-mentioned polymers. The backing on the rim skin adhesive layer may comprise a woven or non-woven fibrous layer, e.g. of polyethylene terephthalate (PET), polyethylene (PE), polypropylene (PP) or any mixture thereof, to provide a soft surface, which may cause less mechanical stress on the skin surfaces in contact with the upper side and/or the circumferential edge of the medical electrode.

The outer diameter of the rim skin adhesive may be in the range of 30 mm to 70 mm such as 40-60 mm for adult sized electrodes or 30-50 mm for electrodes sized for children.

The opening in the rim skin adhesive layer may have a diameter of 10-30 mm, such as 14-24 mm or preferably 16-22 mm, if the rim skin adhesive fully covers the water absorbent skin adhesive, or larger if the rim skin adhesive only partly covers the upper surface of the water absorbent adhesive layer. The overlap between the inner circumference of the rim skin adhesive and the outer diameter of the water absorbent layer may be in the range of 3-10 mm for a small overlap or more for a larger overlap.

### Ion conductive layer

The medical electrode includes an ion conductive layer to ensure electrical contact between the skin and the sensor element, which picks up the biopotential from the person wearing the medical electrode. The ion conductive layer includes an electrolyte. Ions from the electrolyte participate in electrochemical reaction with the sensor. The ion conductive layer converts the biopotential signal from ion polarization to electrons, which are picked up in the sensor element and transferred to the device for monitoring, displaying and/or recording the biopotential.

The ion conductive layer may be a paste, a crosslinked hydrogel or a viscous liquid. "Viscous liquids" are also sometimes referred to as "wet gels". The meaning of the terms "viscous liquid" and "wet gel" is commonly known to a skilled person within the technical area.

The electrolyte contains salts dissolved in the water content in the ion conductive layer. These salts are typically highly soluble chloride salts, such as sodium chloride (NaCI), potassium chloride (KCI) or mixtures thereof.

The water activity of the electrolyte included in the ion conductive layer is important because of the water absorbing properties of the hydrocolloid skin adhesive, which surrounds the ion conductive layer.

Therefore, if the ion conductive layer and the hydrocolloid skin adhesive is in direct contact, the water activity of the electrolyte must be low in order to prevent the adhesive to absorb water from the electrolyte. In such situations, a skin friendly electrolyte gel made of a solid gel are typically used, which may be characterized as being cohesive and having water molecules contained in the polymer or gel matrix, where water does not drip out of the gel. Commercially available examples of such a cohesive skin friendly electrolyte hydrogel is Axelgaard AmGel AG625 and Ludlow hydrogel PR00383.

In variants of the medical electrode, where an ion conductive layer with high water activity is used, it may be necessary to include a water barrier between the ion conductive layer and the hydrocolloid skin adhesive at least during storage of the medical electrode. The water barrier may be a thin lacquer, a foam ring with closed cell structure. Alternatively or additionally, the water barrier may be formed by a protruding element, e.g. a ring shaped rib or protrusion, formed in the release liner, which is dimensioned to extend into a gap formed between the ion conductive layer and the hydrocolloid skin adhesive. The barrier may prevent direct contact between the ion conductive layer during storage of the medical electrode.

The thickness of the ion conductive layer must be so thick that good contact with the skin is ensured throughout the wear time, i.e. also when the thickness of the hydrocolloid skin adhesive is at its maximum thickness, which occurs when the hydrocolloid adhesive is saturated with moisture.

The thickness of the ion conductive layer is typically in the range of 20 to 1500 microns, preferably in the range of 20 to 1100 microns.

### Sensor element

The sensor element may be a printed sensor element or a sensor element with a coating comprising silver (Ag) and silver chloride (AgCl).

The sensor element may be a silver strip coated with AgCl, e.g. as disclosed in US 4524775A and which is currently used in many Ambu BlueSensors.

Alternatively, the sensor element is a printed sensor where the Ag/AgCI sensor printed on an electrically conductive material. An exemplary example of a printed sensor element is described in GB 2341104 B, where an electrically conductive layer is printed on a substrate and the Ag/AgCI layer is printed on the electrically conductive layer.

The substrate is typically a polymer film. Many polymer films are suitable as a substrate for a printed sensor element. Examples of substrates are films based on polyester, for example polyethylene terephthalate (PET), polycarbonate (PC), polyimide (PI) or mixtures comprising one or more of these polymers or laminates or mixtures comprising one or more of these polymers.

The electrically conductive layer printed on the substrate may be an ink, such as a carbon containing ink, e.g. containing graphite or graphene, e.g. in flakes, particles or fibers. Alternatively or additionally, the ink may contain one or more metal particles, flakes or fibers. Suitable metals in an electrically conductive ink include tin, silver, gold, copper, platinum, palladium and alloys or mixtures of particles made of any one of the above-mentioned metals. A particularly preferred ink contains silver, e.g. as silver particles. The electrically conductive layer may be further designed to comprise a track, which extends from the sensor area to the position where a fitting is arranged for connection for establishing electrical connection between the sensor area and the fitting where a cord or wire is attached or where wireless connection means are arranged. Alternatively, the substrate is electrically conductive. The electrically conductive substrate may be formed from a polymer substrate where conductive particulate material is mixed into the polymer matrix to form an electrically conductive substrate. The conductive particulates in the polymer matrix may e.g. be one or more of those used in conductive inks as mentioned above.

It is noted that a sensor element may alternatively be formed on the skin side of a fitting, e.g. a stud, by coating the skin side part of the stud with an AgCl of an Ag/AgCI coating. The fitting or stud may then be aligned with the central opening in the water absorbent skin adhesive, and the ion conductive layer, e.g. by attaching the fitting or stud to the device backing, e.g. as described further below.

### Device backing layer

The medical electrode also comprises a device backing layer. The device backing layer covers at least the sensor element and the ion conductive layer at the upper side of the medical electrode. The device backing layer may further cover the water absorbent adhesive layer either partly or entirely.

The device backing layer is preferably made of an occlusive material. The occlusive material prevents ingress of water into the hydrocolloid adhesive if the medical electrode is worn while taking a shower, which otherwise will result in saturation of the water absorbent adhesive and a potential loss of tackiness, i.e. adhesion to the skin is reduced. Ingress of water into the sensor area may affect the signal quality in a negative way. For example, ingress of water into the ion conductive layer could lower ion concentration in the electrolyte gel and result in higher impedance.

The device backing may be made of for example polyethylene terephthalate (PET), polyethylene (PE), polypropylene (PP), polyimide (PI), polyurethane (PU), thermoplastic polyurethane (TPU) or polycarbonate (PC), or it may be a blend or mixture thereof or a laminate comprising one or more layers, where each layer is made of any one of the above mentioned polymers.

The device backing is attached to the water absorbent skin adhesive and/or the rim skin adhesive to cover and seal the opening in at least the water absorbent skin adhesive. Hereby a sealed compartment around the sensor element and the ion conductive layer is formed to eliminate any escape of water from the ion conductive layer as this could compromise the performance of the medical electrode by an increased impedance caused by a decrease in water activity in the ion conductive layer.

The thickness of the device backing layer must on the one hand be so thick that it protects the sensor element, but on the other hand it must not be so thick that it impedes the freedom of movement of the person wearing the medical electrode. Typically, the thickness of the device backing layer is in the range of 25-100 µm to protect the sensor element.

The device backing layer may be sized to have an overlap with the upper side of the water absorbent skin adhesive and/ or the rim skin adhesive to allow for attachment thereto. The overlap may be 1-5 mm, preferably 1.5-4 mm, more preferred 2-3 mm.

The device backing layer may e.g. be circular, oval or polygonal or more complex shaped, e.g. to correspond to the shape of the medical electrode.

The device backing may have teardrop shape whereby the broad part of the teardrop shape covers the sensor element and the ion conductive layer arranged in the opening in the water absorbent skin adhesive. The fitting may then be arranged at a narrow end of the teardrop form to offset the connection of wires or cords from the sensor element's position.

The narrow end of the teardrop form does preferably not extend beyond the outer circumference of the water absorbent skin adhesive layer and/or the rim skin adhesive. This will allow the patient's skin to be protected from mechanical impact, e.g. scratches, from the fitting, and/or the connector on the wire or cord.

### Fittings

The medical electrode typically comprises fittings for connecting the sensor element to a wired or wireless connection to the device for monitoring, displaying and/or recording the biopotential signals. Such fittings are well-known to the skilled person, and may e.g. be formed as a wire, a snap, a socket for a banana plug, or a stud. The connector may, alternatively, be a part of the device backing layer of the medical electrode, which is formed as a tab onto which a (wired) clip may be attached, a conductor connected to the sensor element or the sensor element may be attached to the underside of the tab for establishing electrical contact between the sensor element and the wired clip.

In a wireless electrode, the fitting may comprise a connection to an electronic circuitry box arranged on the electrode or at a separate patch, which comprises wireless communication means, including, if necessary, means for transferring the analog signals from the medical electrode into digital signals. Additionally, a wireless medical electrode may comprise a plurality, e.g. 2, 3, 4, 5 or more separate sensor elements each arranged in a separate opening in the water absorbent skin adhesive layer and each provided with an ion conductive layer arranged in the opening as described above.

The medical electrode may further comprise a cord, which at its first end is in electrical connection with the sensor element and which at its second end is connectable to a device for monitoring, displaying and/or recording the biopotential signals, e.g. by means of a male or female connector.

The parts of the medical electrode are assembled, e.g. by gluing the parts together. Alternatively, the medical electrode can be assembled using hot melts, or by selecting a welding process which is suitable for use with the materials used, in particular materials used in the device backing and the backing layer(s) of the water absorbent adhesive and/or the rim skin adhesive. The welding methods may e.g. be ultrasonic welding, high frequency welding, laser welding, or hot press where applicable.

### Release liner

Typically, the medical electrode further comprises a release liner to protect the skin side of the medical electrode during storage. The release liner is removed only just before use. A commercially available release liner may be used, e.g. a siliconized film or laminate comprising PET or PE.

A person skilled in the art will appreciate that any one or more of the above-discussed aspects of this disclosure and embodiments thereof may be combined with any one or more of the other aspects or embodiments thereof.

It is noted that the present disclosure is not limited to use exclusively with ECG electrodes and electrocardiography. The suggested solution is equally applicable with all types of biomedical surface electrodes, including biomedical surface electrodes used in neurological studies, such as detecting bioelectrical brain signals, called EEG or electro-encephalography, or detecting bioelectrical signals in muscle tissue, called EMG or electromyography.

The disclosure will now be explained in more detail below by means of examples of non-limiting embodiments with reference to the drawings.
- Figure 1: shows a configuration of the medical electrode according to the disclosure without a rim skin adhesive,
- Figure 2 a-c: show variants of the medical electrode comprising rim skin adhesive with different overlap between the device backing layer, the rim skin adhesive and the water absorbent skin adhesive,
- Figure 3 a-b: show examples of sensor elements that can be used in the present disclosure,
- Figure 4 a-g: show different sizes and shapes of the device backing layer,
- Figure 5 a-e: show different types of fittings that may be used in the present disclosure, and
- Figure 6 a-b: show a simplified prototype as made according to example 1 and used for wear tests in example 2.

In the following, generally, similar elements of different embodiments have been designated by the same reference numerals when shown on different drawings. Thus, a feature discussed in relation to one drawing is not necessarily discussed in relation to all drawings.

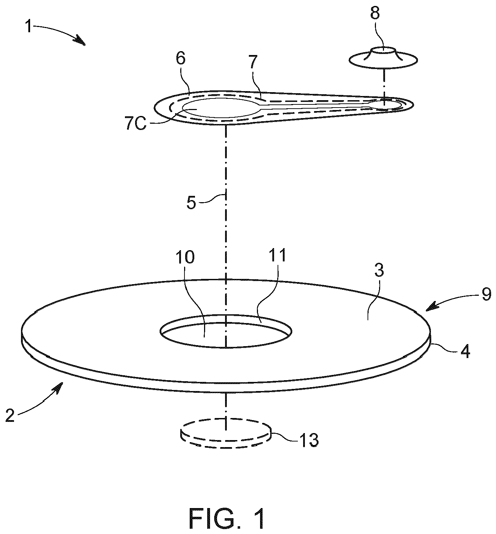

Fig. 1 shows a first example of the biomedical electrode 1 according to the present disclosure.

The medical electrode 1 comprises a water absorbent skin adhesive layer 9 with an opening 10, which in the illustrated embodiment is central in the water absorbent skin adhesive layer 9. The water absorbent skin adhesive layer 9 has a skin side 2 intended for attachment to the skin of a patient and an upper side 3, which faces away from the patient's skin when the medical electrode 1 is applied to a patient. The water absorbent skin adhesive layer 9 may be covered with an occlusive backing, which covers the upper side 3 of the water absorbent skin adhesive layer 9. The occlusive backing may be a separate backing layer attached to the water absorbent skin adhesive layer 9 and/or the backing on the water absorbent skin adhesive layer 9 may be composed of a device backing layer 6, which also covers an area of the electrode with a sensor element 7 and an ion conductive layer 13 as discussed further below.

The water absorbent skin adhesive layer 9 may have a chamfered/beveled face (not shown in figs.) at the outer circumference 4 to reduce any sharp edges, which could be caught by clothing, itch or scratch the patient, e.g. at the surrounding skin or skin which comes into contact with the upper side 3 of the water absorbent skin adhesive layer 9. The beveled face of the edges is also believed to reduce the risk of edge lifting, i.e. when the adhesive slips the skin at the outer circumference 4 of the water absorbent skin adhesive layer 9.

In the drawings the medical electrode 1 is shown as a circular device. It is noted that the skilled person will immediately realize that a medical electrode does not need to be circular. Other shapes are possible, such as oval or elliptical devices or polygonal devices, e.g. square, rectangular hexagonal, octagonal etc. shapes are possible, e.g. with rounded corners. Also, more complex shapes of the medical electrode are possible, such as composed of a combination of a circle, oval or polygon formed integral with a tab like element having e.g. a rectangular or square shape, e.g. with rounded corners, with circular or oval shape.

The water absorbent skin adhesive layer 9 has an opening 10, which is throughgoing and extends from the upper side to the skin side of the layer.

The device backing layer 6 is attached to the upper side 3 of the water absorbent skin adhesive layer 9 and covers the opening 10 in the water absorbent adhesive layer 9. The device backing layer 6 is discussed in further detail below.

The sensor element 7 (outlined in dotted lines in fig. 1) is arranged between the device backing layer 6 and the water absorbent skin adhesive layer 9. The sensor element may be a printed sensor element 7b as illustrated in fig. 1 or an AgCl coated silver strip 7a as discussed further below.

The sensor area 7c of the sensor element 7 is aligned along an alignment axis 5 with the opening 10 in the water absorbent skin adhesive layer 9 and the ion conductive layer 13, which preferably is a solid hydrogel.

The ion conductive layer 13 may fill the opening 10 entirely or partly, e.g. by leaving a gap of 0.5-1.5 mm between the inner circumference 11 of the water absorbent skin adhesive layer 9 and the outer circumference of the ion conductive layer 13.

The ion conductive layer 13 is in direct contact with the sensor area 7c of the sensor element 7, i.e. the AgCl coated part of the silver strip 7a or a Ag/AgCI coated part of the printed sensor element 7b.

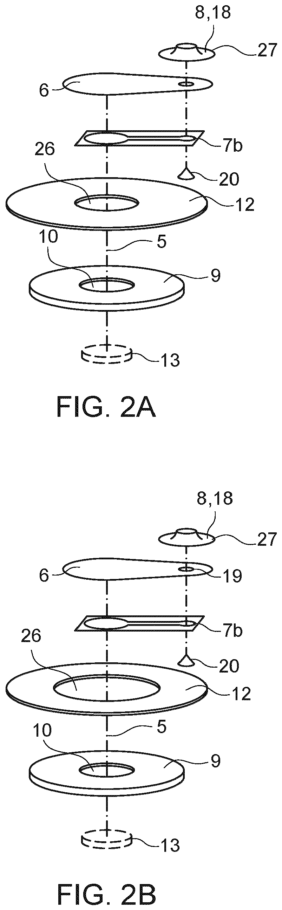

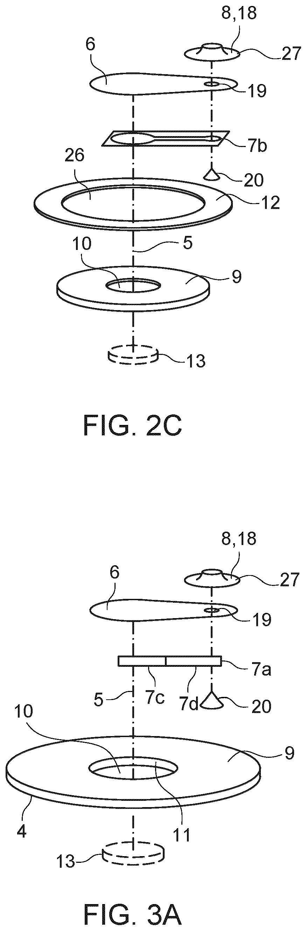

A fitting 8 is attached to the device backing layer 6 and arranged in electrical contact with the sensor element 7. In fig. 1, the fitting shown is a stud, which is typically a two- part stud (see also figs. 2-3), which is used to connect a wire or cord to the medical electrode to transfer bioelectrical signals to a monitor. Alternative fittings or connectors are discussed further below and may be used as alternative to the fitting shown in fig. 1.

Fig. 3a-3b illustrate a similar electrode configuration with different sensor elements. In fig. 3a, the sensor element 7 is a silver strip 7a, which is coated with AgCI on the sensor area 7c. The remaining part of the silver strip acts as a conductor track 7d and extends, e.g. radially, from the central opening 10 to the fitting 8, here exemplified as a stud 18, to establish electrical connection from the sensor area 7c to the fitting 8.

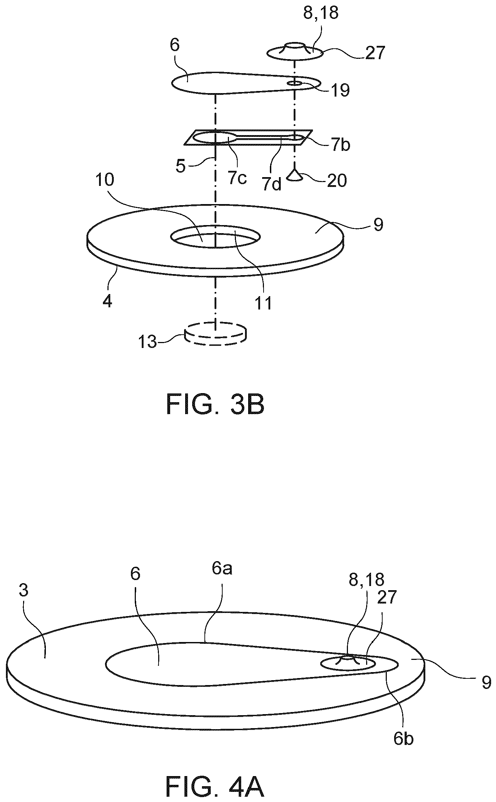

Fig. 3b illustrates a similar configuration where a printed sensor element 7b is shown. The printed sensor element may be a printed sensor element 7b formed on a substrate film as described above. The electrically conductive layer may be further designed to comprise a track forming a printed conductor track 7d, which extends from the sensor area 7c to the position where a fitting 8 is arranged. Thereby the printed conductor track 7d establishes electrical connection between the sensor area 7c and the fitting 8,18 where a cord or wire is attached or where wireless connection means are arranged. The printed sensor element 7b is arranged with the sensor area 7c as well as the electrically conductive layer forming the conductor track 7d directed towards the skin side 2 of the water absorbent skin adhesive layer 9.

Fig. 2a-2c illustrates variants of the medical electrode, which further comprises a rim skin adhesive 12. The rim skin adhesive 12 is arranged to extend radially beyond a circumference of the water absorbent skin adhesive layer 9. The rim skin adhesive is preferred when it is considered necessary to reduce edge lifting. The rim skin adhesive 12 may overlap at least part of the upper side of the water absorbent skin adhesive layer 9. In fig. 2a the rim skin adhesive 12 fully covers the upper side of the water absorbent skin adhesive layer 9 whereby the opening 10 in the water absorbent skin adhesive layer 9 and an opening 26 in the rim skin adhesive 12 have the same diameter and are aligned about the alignment axis 5.

In fig. 2b, the opening 26 in the rim skin adhesive 12 is relatively small compared to the outer circumference of the water absorbent skin adhesive layer 9 and therefore, there is a substantial overlap between the rim skin adhesive 12 and the water absorbent skin adhesive layer 9.

In fig. 2c, this overlap is minimal, such as in the range of 3-10 mm, allowing only for attaching the inner circumference of the rim skin adhesive 12 to the backing on the upper side 3 near the outer circumference of the water absorbent skin adhesive layer 9.

It is noted that although figs. 2a-2c are drawn with a printed sensor element 7b, the medical electrode as shown in figs. 2a-2c may alternatively comprise the AgCl coated silver strip as mentioned above, see e.g. fig. 3a. Also, the fitting 8 shown in figs. 2a-2c are shown as a stud 18, but any suitable fitting 8, e.g. as discussed below, may be used.

In variants of the medical electrode where an ion conductive layer with high water activity or low viscosity, e.g. a wet gel, is used it may be necessary to include a water barrier at or adjacent to the inner circumference 11 of the water absorbent skin adhesive layer 9, i.e. between the ion conductive layer 13 and the (hydrocolloid) water absorbent skin adhesive layer 9 to protect the water absorbent skin adhesive layer 9 from absorbing water from the ion conductive layer 13, at least during storage of the medical electrode 1. The water barrier may be a thin lacquer, a foam ring with closed cell structure or a compartment in the release liner. The barrier is not shown in the figures.

Figs 4a-4g illustrate different examples of shapes of a device backing layer 6 of the medical electrode 1. As already mentioned above, the device backing layer 6 covers at least the opening 10, sensor element 7 and the ion conductive layer 13. The device backing layer 6 may further cover the water absorbent adhesive layer 9 either partly or entirely. The device backing layer 6 may further overlap with the inner circumference of the device rim skin adhesive 12 where applicable. Figs 4a-4c illustrate different sizes of the device backing layer 6 on the medical electrode 1 according to the present disclosure, here exemplified by a medical electrode 1 with a water absorbent skin adhesive layer 9.

As mentioned above, a preferred shape of the device backing layer 6 is the teardrop shape as shown in figs. 4a-4e. The teardrop shaped device backing layer 6 is arranged to cover the sensor element 7 (not seen in in fig. 4a-g), the ion conductive layer 13 (gel) (not seen in in fig. 4a-g) and the opening 10 (not seen in in fig. 4a-g) in the water absorbent skin adhesive layer 9 and/or the rim skin adhesive 12. The broad part 6a of the teardrop shaped device backing layer 6 covers the opening 10 and a part of the upper side 3 of the water absorbent skin adhesive layer 9 and/or the rim skin adhesive 12. The fitting 8 may then be arranged at a narrow part 6b of the teardrop form to offset the connection of wires or cords from the sensor element's 7 position.

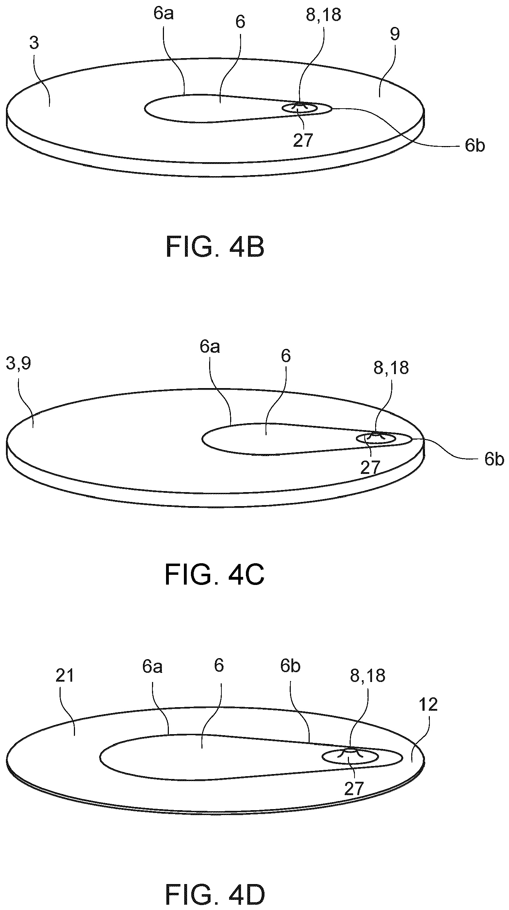

In the exemplified variants of the medical electrode 1 shown in fig. 4a and 4c, the broad part 6a of the teardrop shaped device backing layer 6 extends radially outside the opening 10 and onto the upper side 3 of the medical electrode 1. The narrow part 6b allow attachment of a fitting (exemplified with a stud 18 in fig. 4a and 4c).

In fig. 4b, the broad part of the teardrop shaped device backing layer 6 is minimalized to extend 1-3 mm radially outside the opening 10 and onto the upper surface of the medical electrode 1. The width and length of the narrow part 6b of the teardrop shaped device backing layer 6 is also minimized but still large enough to allow attachment of a fitting (exemplified with a stud 18 in fig. 4b).

The size and/or shape of the device backing layer 6 is dimensioned such that the fitting 8 does not cover the opening 10, neither partially nor entirely, in order to reduce the risk of mechanically induced artifacts caused by a pulling or moving wire/cord attached to the fitting 8.

The length of the device backing layer 6 is dimensioned such that the narrow part 6b which may carry a fitting 8 does not extend beyond the outer circumference 4 of the water absorbent skin adhesive layer 9.

The teardrop shaped device backing's broad part 6a may be attached to the upper side 3 of the water absorbent skin adhesive layer 9 and/or the rim skin adhesive layer 12 to seal the opening 10.

The narrow part 6b of the device backing layer 6 is preferably not attached to the upper side 3 of the water absorbent skin adhesive layer 9 to ease attachment of the wire or cord to the fitting 8 (exemplified with a stud 18 in figs 4a-4e). The narrow part 6b of the teardrop form does preferably not extend beyond the outer circumference of the water absorbent skin adhesive layer 9 and/or the rim skin adhesive 12. This will allow the patient's skin to be protected from mechanical impact, e.g. scratches from edges of the narrow end part of the device backing or from the fitting and/or the connector on the wire or cord, which may be attached thereto.

The upper side 3 of the water absorbent skin adhesive layer 9 exemplified in figs. 4a-c is not covered by a rim skin adhesive 12. The variants shown in figs 4a-c may however comprise a rim skin adhesive 12, e.g. covering some or all of the upper side 3 of the water absorbent skin adhesive, e.g. as illustrated in figs. 2a-2c.

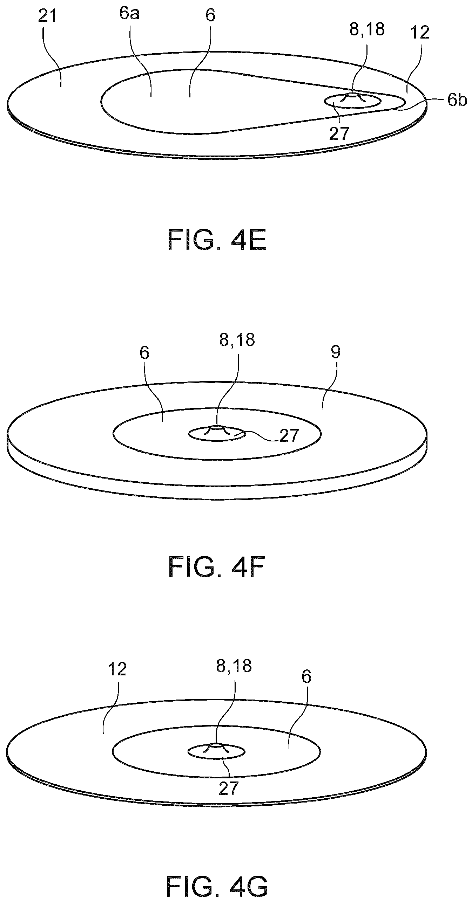

See also figs 4d-4e where the medical electrode 1 is shown with a teardrop shaped device backing layer 6 arranged on the upper side 3 of the water absorbent skin adhesive layer 9 and covers the outer circumference of the water absorbent adhesive layer 9 (not shown in fig. 4d-4e) and a part of the rim skin adhesive layer's upper side 3. In figs. 4d-4e, the teardrop shaped device backing layer 6 also extends to cover a part of the upper side 21 of the rim skin adhesive 12 but to a different degree in fig. 4d and 4e, respectively.

It is noted that a sensor element may alternatively be formed on the skin side of a fitting, e.g. a stud 18, by coating the skin side part of the stud with an AgCI or an Ag/AgCI coating. The fitting 8 or stud 18 may then be aligned with the central opening 10 in the water absorbent skin adhesive, and the ion conductive layer 13, e.g. by attaching the fitting 8 or stud to the device backing layer 6, as shown in fig. 4f or 4g. As also shown in fig. 4f or 4g, this variant may comprise a device backing layer 6 with a geometry similar to the medical electrode's shape, here exemplified with a circular design, although other shapes are possible, e.g. as already discussed above.

Typically, the thickness of the device backing layer 6 may be in the range of 25-100 µm.

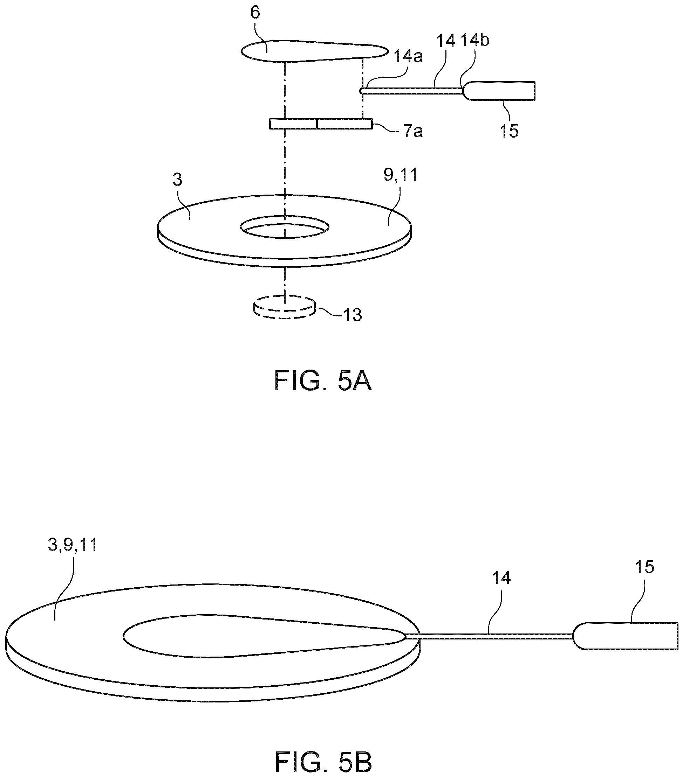

Figs. 5a-5e exemplify a number of different types of fittings 8 or connectors, which are equally applicable in all exemplified variants of the medical electrode 1 discussed above. The fittings 8 or connectors are all intended for connecting the sensor element to a wired or wireless connection to the device for monitoring, displaying and/or recording the biopotential signals.

The fitting shown in figs. 1-3 is a commonly used stud connector 18. The stud 18 has a first part 27 arranged on the top side of the device backing layer 6. A hole 19 is punched in the device backing layer 6 and the connector's second part 20 is inserted into the hole and attached to the first part 18, e.g. by riveting and/or gluing.

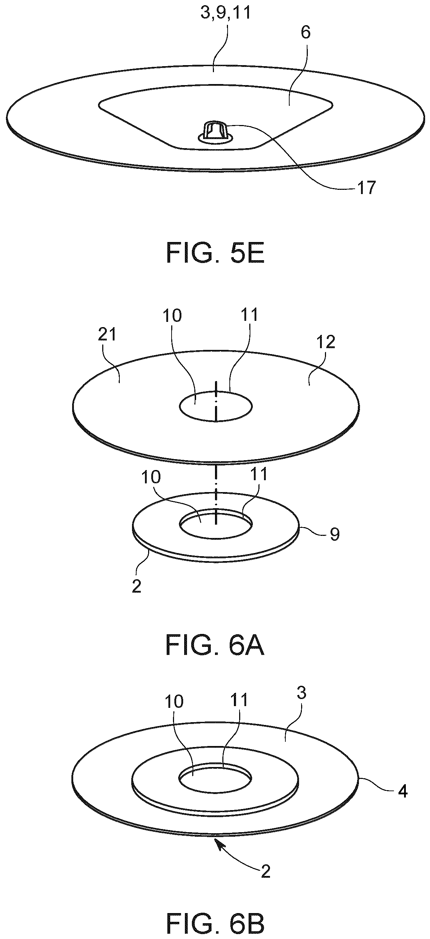

The fitting 8 may, as exemplified in fig. 5e comprise a socket 17 for connecting to a banana plug (not shown).

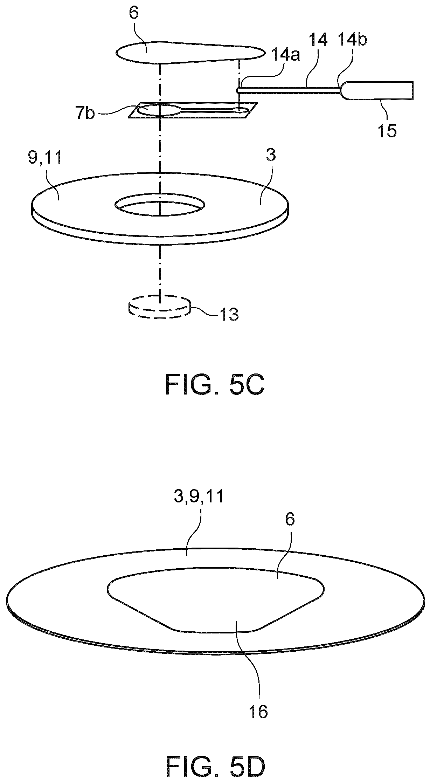

The connector may, as exemplified in fig. 5d be a part of the device backing layer of the medical electrode, which is formed as a tab 16 onto which a wired clip (not shown) may be attached. A conductor track 7d connected to the sensor element or the sensor element 7 itself may be attached to the underside of the tab 16 for establishing electrical contact between the sensor element and the wired clip.

As shown in fig. 5a-5c, the medical electrode 1 may comprise a cord or wire 14, which at its first end 14a is attached in the medical electrode 1 in electrical connection with the sensor element 7. At the second end 14b, the wire or cord 14 is connectable to a device for monitoring, displaying and/or recording the biopotential signals, e.g. by means of a male or female connector 15.

As exemplified in fig. 5a the sensor element 7 may be an AgCI coated silver strip 7a or a printed sensor element 7b as exemplified in fig. 5c.

The parts of the medical electrode are assembled, e.g. by gluing the parts together or by welding as discussed above.

Typically, the medical electrode 1 further comprises a release liner attached to the adhesive skin side 2 of the water absorbent skin adhesive layer 9 during storage. For clarity, the release liner is not shown in the drawings.

### EXAMPLES

### Example 1: Wear test prototypes

8 test samples of the medical electrode according to the present disclosure were made. The device backing, the sensor element and the ion conductive layer (gel electrolyte) and a fitting were omitted for simplicity. All prototypes have a center water absorbent skin adhesive 9 and an acrylic rim skin adhesive 12. Both adhesives are donut shaped with a central opening 10,26 having an inner diameter of 20 mm. The center water absorbent skin adhesive 9 has an outer diameter of 40 mm and the acrylate rim skin adhesive 12 has an outer diameter of 60 mm. The central opening 10,26 in the water absorbent skin adhesive 9 and the rim skin adhesive 12 are aligned so that the rim skin adhesive 12 covers the entire area of the center water absorbent skin adhesive 9. The prototype used is illustrated in fig. 6.

The prototypes have a center water absorbent skin adhesive layer 9 having a total water absorption of 345 mg/cm² and an average thickness of 300 µm. The center water absorbent skin adhesive in the prototypes is exemplified by a commercially available hydrocolloid adhesive MED5094H from Avery Dennison, which has an occlusive backing on the hydrocolloid adhesive of an EVA-PVDC-EVA laminate with a thickness of 95 µm.

The acrylate rim skin adhesive 12 used in the prototype is a Medical tape 1530 from 3M with a non-woven Rayon fiber backing. The rim skin adhesive has a diameter of 60 mm.

### Example 2: Wear test

The electrode prototypes made according to example 1 were subjected to a wear test by 2 healthy human adult volunteers (test subjects).

Four prototypes were placed on the ribs of each test subject to replicate clinical use and relevant skin conditions during wear time of a biomedical electrode according to the present disclosure.

The duration of wear was 7 days unless a prototype fell of or was removed before the end of the wear test.

The prototypes were on average exposed to 8 showers and about one hour of daily exercise, i.e. a total of 7 hours, of moderate to intense sports activities during the entire wear time. This regime represents an activity level in the high end of what adults with the need for ECG monitoring would have as an average activity level. This regime was chosen to ensure that the test subjects had a high production of sweat during their daily exercise.

When prototypes were peeled off either before or at the end of the study, the day of removal and the force required to peel off the prototypes was noted down. In addition, the "ease of removal" was evaluated and given a value on an "ease of removal" scale from 0 to 4, where 0 is characterized as "Very easy" (almost no effort needed before the adhesive detach) and 4 as "Very difficult" (quite a lot of effect needed before the adhesive detach from the skin).

During the wear test, a single prototype was removed on day 5 of the wear test. This prototype was placed laterally on the torso/ribs (on the side of the torso) and released during an excessive stretch where the test subject hung from a bar in the arms. The skin side of the adhesive surface of the prototype was shortly after exposed to water during showering which further compromised the adhesion. The remaining 3 prototypes on that test subject and all 4 prototypes on the second test subject remained attached to the skin until the end of the wear test. The remaining 7 electrodes were all evaluated to be difficult or very difficult to remove corresponding to 3 or 4 on the "ease of removal" scale, which indicates a good adhesion to the skin even after 7 days of exposure to showers, transepidermal water loss (TEWL) and daily perspiration from the test subjects.

The skin was examined immediately after removal of the prototypes and after 3 hours and, if necessary, after 8 hours.

None of the test subjects suffered from skin maceration, irritation or had wounded skin where the prototypes had been attached to the skin.

In conclusion, 7 out of 8 of the prototypes were able to stay on the volunteer's skin for 7 days.

The average wear time for the prototypes was 6.75 days.

### List of reference numbers used in the drawings:

1. Medical electrode
2. Skin side of water absorbent skin adhesive layer
3. Upper side of water absorbent skin adhesive layer
4. Outer circumference of water absorbent skin adhesive layer
5. Alignment axis
6. Device backing layer
   a. Broad part
   b. Narrow part
7. Sensor element
   a. AgCI coated silver strip
   b. Printed sensor element
   c. Sensor area
   d. Conductor track
8. Fitting (Connector)
9. Water absorbent skin adhesive layer
10. Opening in water absorbent skin adhesive layer
11. Inner circumference of water absorbent skin adhesive layer
12. Rim skin adhesive
13. Ion conductive layer (electrolyte)
14. Wire, cord
   a. 1^{st} end
   b. 2^{nd} end
15. Male or female connector
16. Tab
17. Socket, for banana plug
18. Stud
19. Hole in device backing layer
20. Second part of stud
21. Upper side of rim skin adhesive
26. Opening rim skin adhesive
27. First part of stud

## Claims

1. A medical electrode for long-term biopotential monitoring and/or recording of biopotential signals, said medical electrode comprising
- a sensor element,
- a device backing,
- an ion conductive layer in contact with the skin side of the sensor element, and
- a water absorbent skin adhesive layer surrounding the ion conductive layer and having an upper side and a skin side adapted to attach to skin,
wherein said water absorbent skin adhesive has a total water absorption capacity of at least 200 mg water/cm².

2. The medical electrode according to claim 1, wherein the water absorbent skin adhesive is a hydrocolloid skin adhesive.

3. The medical electrode according to claim 1 or 2, wherein the water absorbent skin adhesive has a total water absorption capacity of at least 50 mg water/cm² per day for at least 4 days.

4. The medical electrode according to any one of the preceding claims, wherein said medical electrode is adapted for a wear time of at least 4 days, preferably at least 5 days, even more preferred at least 7 days.

5. The medical electrode according to any one of the preceding claims, wherein the water absorbent skin adhesive has a total water capacity of
- at least 200 mg water/cm² during the first four days of wear time, or
- at least 250 mg water/cm² during the first five days of wear time, or
- at least 300 mg water/cm² during the first six days of wear time, or
- at least 350 mg water/cm² during the first seven days of wear time, or
- at least 400 mg water/cm² during the first eight days of wear time, or
- at least 450 mg water/cm² during the first nine days of wear time, or
- at least 500 mg water/cm² during the first ten days of wear time, or
- at least 550 mg water/cm² during the first eleven days of wear time, or
- at least 600 mg water/cm² during the first twelve days of wear time, or
- at least 650 mg water/cm² during the first thirteen days of wear time, or
- at least 700 mg water/cm² during the first fourteen days of wear time.

6. The medical electrode according to any one of the claims 2 to 5, wherein the polymer matrix of the hydrocolloid skin adhesive comprises one or more polymer(s) selected from the group consisting of polyisobutylene, styrene-isoprene-styrene, ethylene-vinyl acetate, butyl rubber, acrylate and silicone and any mixture thereof, and/or
where the hydrocolloid particles in the hydrocolloid skin adhesive are selected from the group consisting of carboxymethyl cellulose, hydroxyethyl cellulose, cetyl hydroxyethyl cellulose, polyacrylic acid, pectin, locust bean gum, gum karaya and guar gum and any mixtures thereof.

7. The medical electrode according to any one of the claims 2 to 6, wherein the amount of hydrocolloid particles in the hydrocolloid skin adhesive is at least 20 wt% based on total weight of the hydrocolloid skin adhesive, such as in the range of 20-60 wt% based on total weight of the hydrocolloid skin adhesive.

8. The medical electrode according to any one of the preceding claims, wherein the ion conductive layer is a paste, a crosslinked hydrogel or a viscous liquid.

9. The medical electrode according to any one of the preceding claims, further comprising a rim skin adhesive extending beyond a circumference of the water absorbent skin adhesive, wherein said rim skin adhesive is a different type of skin adhesive than the water absorbent skin adhesive and wherein said rim skin adhesive optionally overlaps at least part of the upper side of the water absorbent skin adhesive.

10. The medical electrode according to any one of the preceding claims, wherein the thickness of the ion conductive layer is in the range of 20 to 1500 microns, preferably in the range of 20 to 1100 microns,
and/or where the thickness of the water absorbent skin adhesive layer is in the range of 150 to 1500 microns, preferably in the range of 300 to 1000 microns.

11. The medical electrode according to any one of the preceding claims, wherein the sensor element is a printed sensor element or a sensor element with a coating comprising silver (Ag) and silver chloride (AgCl), or where the sensor element is a metal electrode in the form of a silver (Ag) strip coated with silver chloride (AgCl).

12. The medical electrode according to any one of the preceding claims, wherein the device backing covers at least the sensor element and the ion conductive layer.

13. The medical electrode according to claim 12, wherein said device backing further covers the water absorbent adhesive layer partly or entirely.

14. The medical electrode according to any one of the preceding claims, wherein said device backing is made of an occlusive material.

15. The medical electrode according to any one of the preceding claims, wherein the water absorbent skin adhesive layer further comprises a separate occlusive backing on the upper side.

16. The medical electrode according to any one of the preceding claims, further comprising one or more fittings for connecting the sensor element to a wired or wireless connection to a device for monitoring, displaying and/or recording the biopotential signals, and wherein the fittings optionally is formed as a wire, a snap, a socket, such as for a banana plug, or a tab.

17. The medical electrode according to any one of the preceding claims, further comprising a cord which at its first end is in electrical connection with the sensor element and which at its second end is connectable to a device device for monitoring, displaying and/or recording the biopotential signals.
